# EUROPEAN PATENT SPECIFICATION

(11) **EP 3 818 985 B1**
(45) Date of publication and mention of the grant of the patent: **05.01.2022**
(21) Application number: 20460040.7
(22) Date of filing: 21.10.2020
(51) Int. Cl.: A61K 35/747, A61P 1/00, A61P 1/04, A61P 31/00, A61P 31/04

(54) **LACTOBACILLUS FERMENTUM PL9 AND ITS APPLICATION**
LACTOBACILLUS FERMENTUM PL9 UND SEINER ANWENDUNG
LACTOBACILLUS FERMENTUM PL9 ET SON APPLICATION

(30) Priority: 06.11.2019 PL 43172219
(43) Date of publication of application: 12.05.2021
(73) Proprietor: Sanprobi Spólka Z Ograniczona Odpowiedzialnoscia Spólka Komandytowa, 70-535 Szczecin (PL)
(72) Inventor: Heczko, Piotr, 31-517 Kraków (PL); Strus, Magdalena, 30-617 Kraków (PL)
(74) Representative: Magonska, Alina

(56) References cited:
- KR-A- 20190 060 271
- US-A1- 2016 348 191
- MAR?A SALAS-JARA ET AL: "Biofilm Forming Lactobacillus: New Challenges for the Development of Probiotics", MICROORGANISMS, vol. 4, no. 4, 20 September 2016 (2016-09-20), page 35, XP055431439, DOI: 10.3390/microorganisms4030035
- AOUDIA NABIL ET AL: "Biofilms ofLactobacillus plantarumandLactobacillus fermentum: Effect on stress responses, antagonistic effects on pathogen growth and immunomodulatory properties", FOOD MICROBIOLOGY, ACADEMIC PRESS LTD, LONDON, GB, vol. 53, 29 April 2015 (2015-04-29), pages 51-59, XP029316563, ISSN: 0740-0020, DOI: 10.1016/J.FM.2015.04.009

<110> Sanprobi Sp. z o.o. Sp. komandytowa
<120> A new strain of Lactobacillus fermentum PL9 and its application
<140> 20460040.7-1112
   <141> 2020-10-21
<150> T.431722
   <151> 2019-11-06
<160> 2
<210> 1
   <211> 1450
   <212> DNA
   <213> Lactobacillus fermentum
<400> 1
<210> 2
   <211> 419
   <212> DNA
   <213> Lactobacillus fermentum
<400> 2
   gggcgcaagc ctgatggagc aacaccgcgt gagtgaagaa gggtttcggc tcgtaaagct      60
   ctgttgttaa agaagaacac gtatgagagt aactgttcat acgttgacgg tatttaacca     120
   gaaagtcacg gctaactacg tgccagcagc cgcggtaata cgtaggtggc aagcgttatc     180
   cggatttatt gggcgtaaag agagtgcagg cggttttcta agtctgatgt gaaagccttc     240
   ggcttaaccg gagaagtgca tcggaaactg gataacttga gtgcagaaga gggtagtgga     300
   actccatgtg tagcggtgga atgcgtagat atatggaaga acaccagtgg cgaaggcggc     360
   tacctggtct gcaactgacg ctgagactcg aaagcatggg tagcgaacag gattagata     419
<110> Sanprobi Sp. z o.o. Sp. komandytowa
<120> A new strain of Lactobacillus fermentum PL9 and its application
<140> 20460040.7-1112
   <141> 2020-10-21
<150> T.431722
   <151> 2019-11-06
<160> 3
<210> 1
   <211> 41
   <212> DNA
   <213> Starters
<400> 1
   gccgcctaag gtgggacaga tctgatcgta gatcagtcaa g 41
<210> 2
   <211> 1450
   <212> DNA
   <213> Lactobacillus fermentum
<400> 2
<210> 3
   <211> 419
   <212> DNA
   <213> Lactobacillus fermentum
<400> 3
   gggcgcaagc ctgatggagc aacaccgcgt gagtgaagaa gggtttcggc tcgtaaagct     60
   ctgttgttaa agaagaacac gtatgagagt aactgttcat acgttgacgg tatttaacca    120
   gaaagtcacg gctaactacg tgccagcagc cgcggtaata cgtaggtggc aagcgttatc    180
   cggatttatt gggcgtaaag agagtgcagg cggttttcta agtctgatgt gaaagccttc    240
   ggcttaaccg gagaagtgca tcggaaactg gataacttga gtgcagaaga gggtagtgga    300
   actccatgtg tagcggtgga atgcgtagat atatggaaga acaccagtgg cgaaggcggc    360
   tacctggtct gcaactgacg ctgagactcg aaagcatggg tagcgaacag gattagata     419

## Description

The present invention is defined in claims 1-6. The subject of the invention is a new strain of *Lactobacillus fermentum* PL9 and its use for protection against pathogens and for increasing the synthesis of tight junction proteins within the intestinal epithelium and for preventing infections, as well.

Human health is highly dependent on the appropriate composition of the intestinal microbiota and its functioning in close association with the intestinal epithelial cells and the local immune system. Such a connection ensures homeostasis of the whole organism. The bacteria that make up the intestinal microbiota adhere to the intestinal epithelial cells by binding to mucus mucins and colonize the mucous membrane surface by competing with pathogens penetrating from the outside into the gastrointestinal tract and interacting with the factors and cells of the specific and non-specific immune system, which then protects the organism against diseases by identifying and eliminating the pathogens. Therefore, the proper functioning of the immune system is significantly influenced by the composition of the bacterial flora in the digestive system. Bacteria adhering to the mucosa also have many regulatory functions, influencing the functioning of intestinal mucosa cells and many others. Among other things, they influence the production of intercellular junction proteins. The intestinal mucosa ensures the absorption of water, electrolytes, and food ingredients. Its integrity is ensured primarily by the so-called tight junctions, consisting of different proteins. Tight junctions also protect the gastrointestinal tract and the organism against the penetration of virulent pathogens: bacteria, viruses and fungi into the submucosa and the intestinal lymphatic system, which can lead to generalized infection.

Thus, disturbances in the composition of the intestinal microbiota have numerous dangerous consequences for the functioning of the intestine and the entire organism. Many factors, but most of all pathogens: bacteria, viruses and fungi, antibiotic therapy, and improper diet, are the causes of disturbances in the composition of the gastrointestinal tract microbiota. There is a lot of evidence, including clinical evidence, that the use of appropriate probiotic bacteria leads to the amelioration of the composition of the gut microbiota and the improvement of health.

It is known from an article "Biofilm Forming Lactobacillus: New Challenges for the Development of Probiotics" authored by Salas-Jara et al. (MICROORGANISMS, vol. 4, no. 4, 20 September 2016) That a necessary condition prior to colonization by L.fermentum is the preferential adhesion to the intestinal or oral mucosa, aiding formation of the biofilm and to exclude, by competitve inhibition, pathogenic bacteria. The biofilm of L.fermentum with other with other Lactobacillus is able to potentiate probiotic capacity because they resist gastrintestinal conditions, they are immunomofdulators and inhibit the growth of pathogens.

It is known from AOUDIA, N. ET AL.: "Biofilms of Lactobacillus plantarum and Lactobacillus fermentum: Effect on stress responses, antagonistic effects on pathogen growth and immunomodulatory properties", (FOOD MICROBIOLOGY, vol. 53, 29 April 2015 (2015-04-29), pages 51 - 59), that Lactobacillus fermentum strains are able to grow a biofilms on abiotic surfaces, and that TNF-α production by LPS activated human monocytoid cells was supressed by supernatants from Lactobacillus cultivated as biofilms, but only Lactobacillus fermentum NA4 showed anti-inflammatory effects in zebrafish embryos fed with probiotic bacteria, as assessed by cytokine transcript level ( TNF-α, IL-1β and IL-10).

There is a composition containing *Lactobacillus fermentum* 57A, known from the Polish patent specification of Pat.210465, which has a very good affinity for the intestinal epithelium and has the ability to adhere to this monolayer.

There is an antiallergic agent, known from the Polish patent specification of Pat.204931, which as an active ingredient contains strains of the genus *Lactobacillus,* including those belonging to the species *Lactobacillus fermentum.* According to the invention, the agent composition contains *Lactobacillus fermentum* CP34. The use of an agent according to the invention contributes to a decrease in the level of IgE antibodies in the blood.

There is a new *Lactobacillus fermentum* KBL375 strain, known from the patent specification of WO 2019103198, which is isolated from the human gastrointestinal tract. This strain has immunoregulatory properties and helps heighten intestinal wall integrity.

There is a pharmaceutical composition, known from the patent description of CA 3018035, containing the *Lactobacillus fermentum* LF05 strain deposited under the number DSM 32277, intended for the treatment of vaginal infections. The Applicant has selected a strain of the *Lactobacillus fermentum* species, which was found to be active and effective against the pathogens *Candida albicans* and *Gardnerella vaginalis.*

There is a group of *Lactobacillus fermentum* species, known from the patent specification of KR 101452234, isolated from people living in the Korean village of longevity. These strains are characterized by excellent adhesion ability to gastric epithelial cells and high gastric acid and bile resistance.

The aim of the invention was to select a new strain from the collection of bacteria strains of the genus *Lactobacillus,* which apart from the characteristics of this genus and probiotic properties, would have a high degree of ability to stimulate the production of tight junction proteins on the example of occludin, while showing a very high affinity for the mucus covering the intestinal mucosa and the associated ability to colonize through biofilm formation. Such a strain could be used to correct the microbiota composition and to regulate the permeability of the epithelium, thus extinguishing inflammation in the gastrointestinal tract.

The essence of the invention is a new strain of *Lactobacillus fermentum* designated by the symbol PL9, which has been deposited in accordance with the Budapest Treaty on the International Recognition of the Deposit of Microorganisms for the Purposes of Patent Procedure at the Polish Collection of Microorganisms (PCM), located at the Institute of Immunology and Experimental Therapy in Wroclaw (53-114 Wroclaw, Rudolfa Weigla 12). The new *Lactobacillus fermentum* PL9 strain was lodged on August 13^{th}, 2018 and the number of B/00109 was assigned.
The *Lactobacillus fermentum* PL9 strain was isolated from a healthy person with normal intestinal microbiota, from the Polish population. Due to its human origin, the *Lactobacillus fermentum* PL9 strain has outstanding adherence properties to the intestinal epithelium in humans.

The *Lactobacillus fermentum* PL9 strain meets the WHO/FAO criteria determining the affiliation of strains of bacteria to probiotics and has the GRAS (Generally Recognized as Safe) status, and it has been thoroughly tested for antibiotic resistance. It has been shown that this strain shows a range of antibiotic resistance typical of the genus and species. Since it does not have antibiotic resistance mechanisms, and therefore does not constitute a reservoir of drug resistance genes in the natural microbiome, it can be safely used in humans.

The new *Lactobacillus fermentum* PL9 strain has the high ability to stimulate the production of occludin by intestinal cells.

The new *Lactobacillus fermentum* PL9 strain has the ability to form biofilm.

The new *Lactobacillus fermentum* PL9 strain has the ability to adhere to mucins.

Herein described is the use of a new *Lactobacillus fermentum* PL9 strain deposited at the Polish Collection of Microorganisms under the number of B/00163 for the production of an agent improving the intestinal barrier function and preventing infections by improving the microbiota composition due to the elimination of pathogens.

The present invention is illustrated in the drawing, in which:
Fig. 1 shows the result of the API 50 CH test,
Fig. 2 shows a photo of the PCR reaction with Lactobacillus fermentum - Paths: 1 - *Lactobacillus fermentum* PL9 strain; 2 - negative control; 3 - positive control (*Lactobacillus fermentum* ATCC 9338 strain), M - size marker
Fig. 3 shows the resistance of the *Lactobacillus fermentum* PL9 strain to bile salts at concentrations of 1.2.5.10.20 g / l,
Fig. 4 shows the adherence of the *Lactobacillus fermentum* PL9 strain to the Caco-2 intestinal epithelium line,
Fig. 5 shows the stimulation of the production of tight junction proteins (occludins) in the intestinal epithelium by the *Lactobacillus fermentum* PL9 strain,
Fig. 6 shows the antagonistic activity of the *Lactobacillus fermentum* PL9 strain towards selected species of bacteria and pathogenic fungi,
Fig. 7 shows the ability of the *Lactobacillus fermentum* PL9 strain to produce biofilm,

The invention also covers the use of a new *Lactobacillus fermentum* PL9 strain deposited at the Polish Collection of Microorganisms under the number of B/00163 for use in the prevention of pathogenic fungi and bacterial infections in the human gastrointestinal tract, by correcting microbiota composition due to pathogens elimination, and also for use in improving intestinal barrier function.

The present invention is illustrated in the drawing, in which:

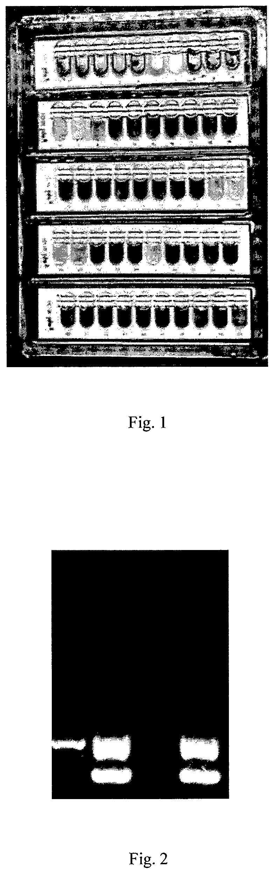

Fig. 1 shows the result of the API 50 CH test,
Fig. 2 shows a photo of the PCR reaction with Lactobacillus fermentum - Paths: 1 - *Lactobacillus fermentum* PL9 strain; 2 - negative control; 3 - positive control (*Lactobacillusfermentum* ATCC 9338 strain), M - size marker

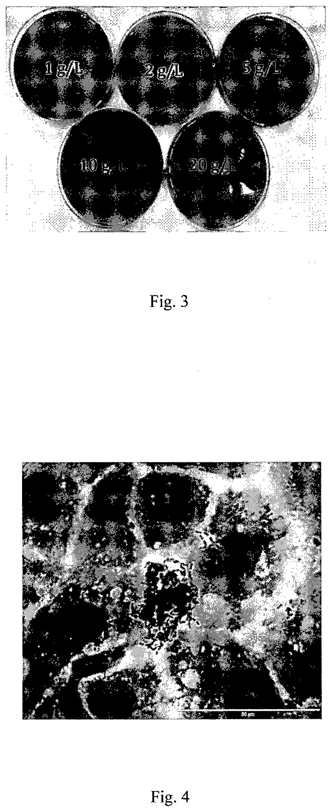

Fig. 3 shows the resistance of the *Lactobacillus fermentum* PL9 strain to bile salts at concentrations of 1.2.5.10.20 g / l,
Fig. 4 shows the adherence of the *Lactobacillus fermentum* PL9 strain to the Caco-2 intestinal epithelium line,

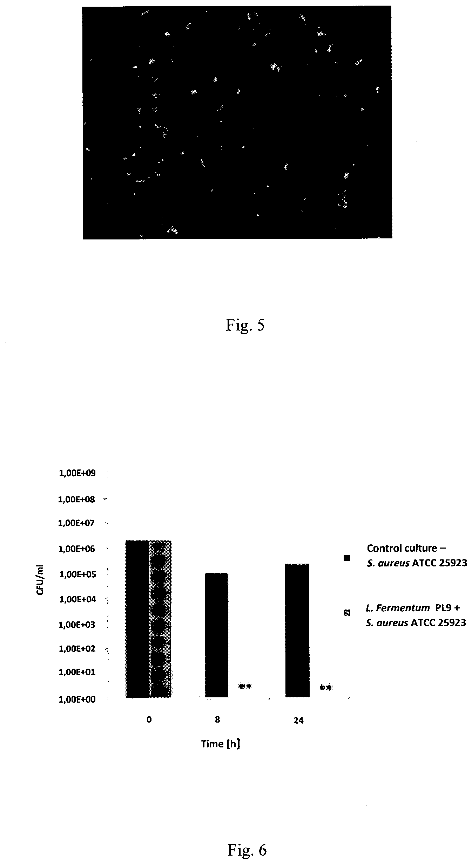

Fig. 5 shows the stimulation of the production of tight junction proteins (occludins) in the intestinal epithelium by the *Lactobacillus fermentum* PL9 strain,

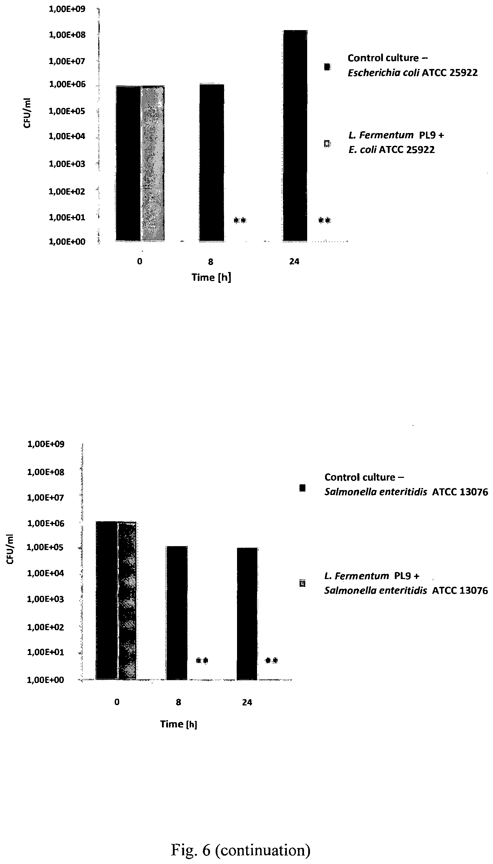

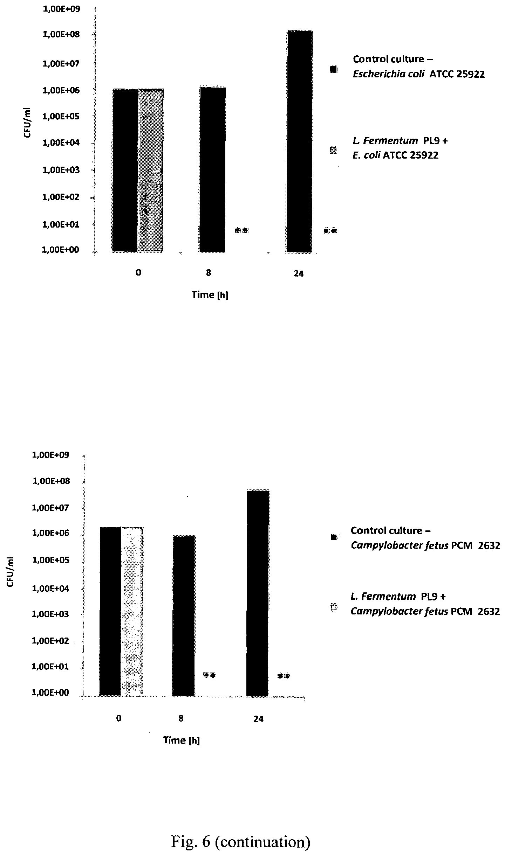

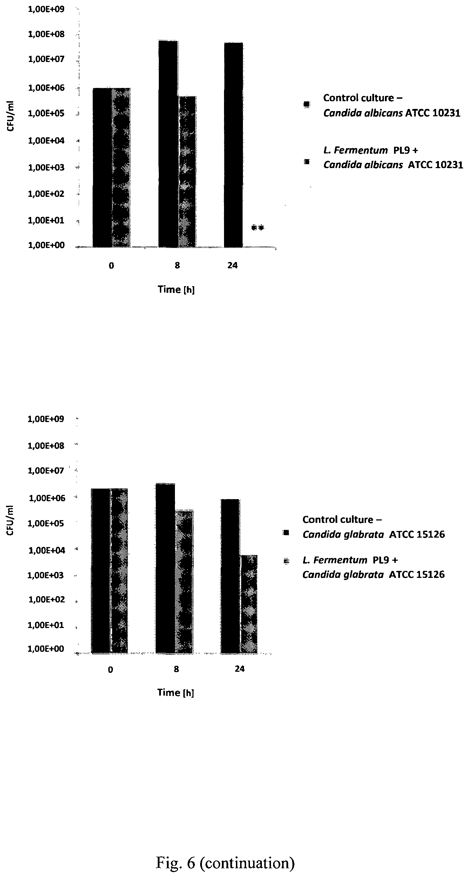

Fig. 6 shows the antagonistic activity of the *Lactobacillus fermentum* PL9 strain towards selected species of bacteria and pathogenic fungi,

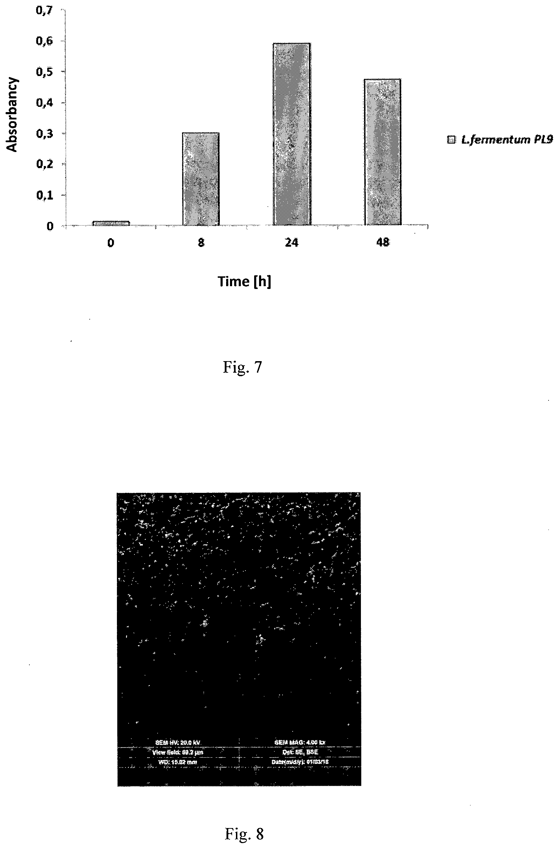

Fig. 7 shows the ability of the *Lactobacillus fermentum* PL9 strain to produce biofilm,
Fig. 8 shows the image (in a scanning electron microscope) of the biofilm that is produced by the *Lactobacillus fermentum* PL9 strain during the 48 hour cultivation.

### I. Phenotypic identification of the PL9 strain using the bioMerieux API^{®}50 CH kit.

| | | |
|---|---|---|
| 10 | D-galactose | + |
| 11 | D-glucose | + |
| 12 | D-fructose | + |
| 13 | D-mannose | - |
| 14 | L-sorbose | - |
| 15 | L-rhamnose | - |
| 16 | Dulcitol | - |
| 17 | Inositol | - |
| 18 | D-mannitol | - |
| 19 | D-sorbitol | - |
| 20 | Methyl-αD- mannopyranoside | - |
| 21 | Methyl-αD- glucopiranoside | - |
| 22 | N-acetyl-glucosamine | - |
| 23 | Amygdalin | - |
| 24 | Arbutin | - |
| 25 | Exulin Ferric citrate | - |
| 26 | Salicin | - |
| 27 | D-cellobiose | - |
| 28 | D-maltose | + |
| 29 | D-lactose (bovine) | + |
| 30 | D-melibiose | + |
| 31 | D-saccharose | + |
| 32 | D-trehalose | - |
| 33 | Inulin | - |
| 34 | D-melesitosis | - |
| 35 | D-raffinose | + |
| 36 | Starch | - |
| 37 | Glycogen | - |
| 38 | Xylitol | - |
| 39 | Gentiobiose | - |
| 40 | D-turanose | - |
| 41 | D-lyxose | - |
| 42 | D-tagatose | - |
| 43 | D-fucose | - |
| 44 | L-fucose | - |
| 45 | D-arabitol | - |
| 46 | L-arabitol | - |
| 47 | Potassium gluconate | + |
| 48 | Potassium 2-ketogluconate | - |
| 49 | Potassium 5-ketogluconate | + |

The result of the API 50 CH test for the PL9 strain.

On the basis of the obtained biochemical profile, the apiWeb program identified the PL9 strain as *Lactobacillus fermentum.*

### II. Identification of the Lactobacillus fermentum PL9 strain with the use of the PCR method (Polymerase Chain Reaction)

### II.1. Isolation of genomic DNA of bacteria:

The DNA GeneMATRIX Basic DNA Purification Kit (EURx), which uses the ability of DNA to bind to silica deposits in high concentrations of chaotropic salts, was used to isolate genomic DNA.

The DNA isolation procedure was as follows:
1. A 24-hour bacterial cell culture grown in liquid MRS medium (Oxoid) was centrifuged in a volume of 1 ml (2 min., 12,000 rpm).
2. The supernatant was removed and the sediment was thoroughly resuspended in 250 µl of Cell R Suspension Buffer (included in the kit) with addition of 200 µl of Lysis Blue lysis buffer (included the kit) until a homogeneous blue suspension was obtained.
3. 350 µl of Neutral B neutralizing buffer (included in the kit) was added to the cell suspension. The contents of the test tubes were thoroughly and slowly mixed by means of repeated inversion until the blue color of the suspension was completely gone.
4. After the lysis was completed, the mixture was centrifuged (7 min., 12,000 rpm), and the filtrate was placed on a minicolumn with a special silica deposit and centrifuged again (1 min., 12,000 rpm).
5. The deposit was washed twice: first with 500 µL of Wash UX1 washing buffer, then with 650 µL of Wash UX2 washing buffer (included in the kit).
6. The purified DNA was eluted from the deposit with 50 µl of Elution buffer (included in the kit), heated to 80°C.
7. The isolated DNA was stored in an Eppendorf test tube at temperature of 4°C until further analysis.

### II.2 Polymerase chain reaction (PCR):

Table 2 shows the sequences of the primers used and the sizes of the corresponding amplicons.

**Tab. 2. Primer sequences and size of the amplicons of the sought species of bacteria.**

| Primer | Sequence 5'→3' | Species | Size of amplicons |
|---|---|---|---|
| Lfpr FermII | | *L. fermentum* | 300bp and 500bp |

The amplification program for the species *Lactobacillus fermentum* was carried out in the S 1000 thermocycler (BioRad) and was as follows:

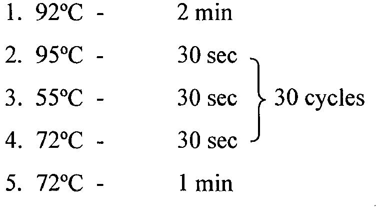

Amplification was performed according to the method of Walter et al. (Walter J, Tannock GW, Tilsala-Timisjarvi A, Rodtong S, Loach DM, Munro K, Alatossava T. Detection and identification of gastrointestinal Lactobacillus species by using denaturing gradient gel electrophoresis and species-specific PCR primers. Appl Environ Microbiol. 2000;66(1):297-303). The primer sequences used are also derived from this publication.

After the PCR reaction was completed, the amplification products were analyzed in a 1.5% agarose gel stained with ethidium bromide. The samples were applied to the gel wells in a volume of 7 µl with the addition of 3 µl of loading buffer. The electrophoresis was carried out in the TBE buffer at a concentration of 0.5x, for 1.5 hours, at a voltage of 80V, in an electrophoresis apparatus (BioRad).

The image of the gel was viewed using the GelDoc XR + system (BioRad), which included a UV transilluminator, an image-collecting camera, and an Image Lab (BioRad) computer program for image documentation and analysis.

The presence of a PCR product with the appropriate number of base pairs was considered a positive reaction result. Moreover, a negative control, which was distilled water added to the reaction buffer instead of genomic DNA of bacteria as well as a positive control, which was an amplification product obtained with the use of the standard strain DNA and a band size marker (GeneRuler 100bp, Axygen) were placed on each gel.

Fig. 2 shows a photo of the PCR products of *Lactobacillus fermentum*; lanes: 1-(PL9 strain); 2-negative control; 3-positive control (*Lactobacillus fermentum* ATCC 9338 strain); M - size marker.

### III. Identification of the Lactobacillus fermentum PL9 strain using 16S rRNA sequencing.

DNA amplicons obtained in the PCR reaction were separated by electrophoresis in the presence of dyes and a DNA ladder. The results were visualized using an archiver. Amplicons were subjected to DNA sequencing and the obtained sequences were analyzed *in silico* using NCBI Blast tools. The correctness of the read sequences was verified on the basis of visual inspection and an analysis of the NCBI Genbank database.

**Tab. 3. Tabulated list of DNA sequences obtained for the tested PL9 strain with the use of literature-based oligonucleotides, enabling the species identification of the bacterial strains.**

| **Strain name** | **PL9** |
|---|---|
| DNA sequence 5' - 3' primers 1 | |
| | |
| | |
| DNA sequence 5'-3' primers 2 | |

On the basis of the obtained DNA sequences and the analysis of the NCBI Genbank database, the PL9 strain was identified as *Lactobacillus fermentum.*

### IV. Determination of antibiotic sensitivity of the Lactobacillus fermentum PL9 strain

### a. Antibiotic resistance tested using the disk diffusion method.

In order to test the drug resistance of the *L. fermentum* PL9 strain by the disc diffusion method, the culture suspension (OD = 0.5 McFarland) was spread on the MRS agar plate (Oxoid) in a Petri dish. The substrate with the suspension applied was then left at room temperature for 15 minutes to dry. After this time, discs impregnated with appropriate antibiotics were applied onto the plate with sterile tweezers. Incubation was carried out under anaerobic conditions for 24 hours at temperature of 37°C. After incubation, the zones of inhibited growth of the bacterial strain around the antibiotic disc (in mm) were measured. Interpretation of drug resistance, which differentiated between the *L. fermentum* PL9 strain that was susceptible to and resistant to the tested antibiotics, was made in accordance with EUCAST. The following antibiotics were used in the experiment: penicillin, ciprofloxacin, cefaclor, doxycycline, oxacillin, co-trimoxazole, erythromycin, ampicillin, clindamycin, vancomycin, gentamicin, metronidazole (Oxoid).

**Tab. 4. The size of the zones of growth inhibition obtained for the tested L. fermentum PL9 strain and the reference strain of L. fermentum ATCC 9338.**

| Antibiotic Concentration | *L. fermentum* PL9 | *L. fermentum* ATCC 9338 |
|---|---|---|
| Penicillin (P) 1 µg | 15 mm | 14 mm |
| Ciprofloxacin (CIP) 5 µg | 14 mm | 11 mm |
| Cefaclor (CEC) 30 µg | 6 mm | 6 mm |
| Doxycycline (DO) 30 µg | 27 mm | 30 mm |
| Oxacillin (OX) 1 µg | 6 mm | 6 mm |
| Co-trimoxazole (SXT) 25 µg | 6 mm | 8 mm |
| Erythromycin (E) 15 µg | 35 mm | 30 mm |
| Ampicillin (AMP) 2 µg | 16 mm | 11 mm |
| Clindamycin (DA) 2 µg | 35 mm | 29 mm |
| Gentamicin (CN) 30 µg | 16 mm | 15 mm |
| Vancomycin (VA) 30 µg | 6 mm | 6 mm |
| Metronidazole (MTZ) 5 µg | 6 mm | 6 mm |

Using the qualitative method it was shown that the PL9 strain's resistance/susceptibility to antibiotics was consistent with the species pattern.

### b. Antibiotic resistance determined by the quantitative method in compliance with EFSA's interpretation data using the E-tests.

In order to test drug resistance of probiotic strains by E-tests, the culture suspension (OD = 0.5 McFarland) was spread on the MRS agar plate (Oxoid) in a Petri dish. The substrate with the suspension applied was then left at room temperature for 15 minutes to dry. After this time, E-test strips impregnated with antibiotics of various concentrations were applied onto the plate with sterile tweezers. The plates incubation was carried out under anaerobic conditions for 24 hours at temperature 37 °C. After incubation, the MIC values (mg/L) were read. The following antibiotics were used in the experiment: ampicillin, gentamicin, tetracycline, clindamycin, erythromycin, chloramphenicol, vancomycin, kanamycin, streptomycin (Oxoid).

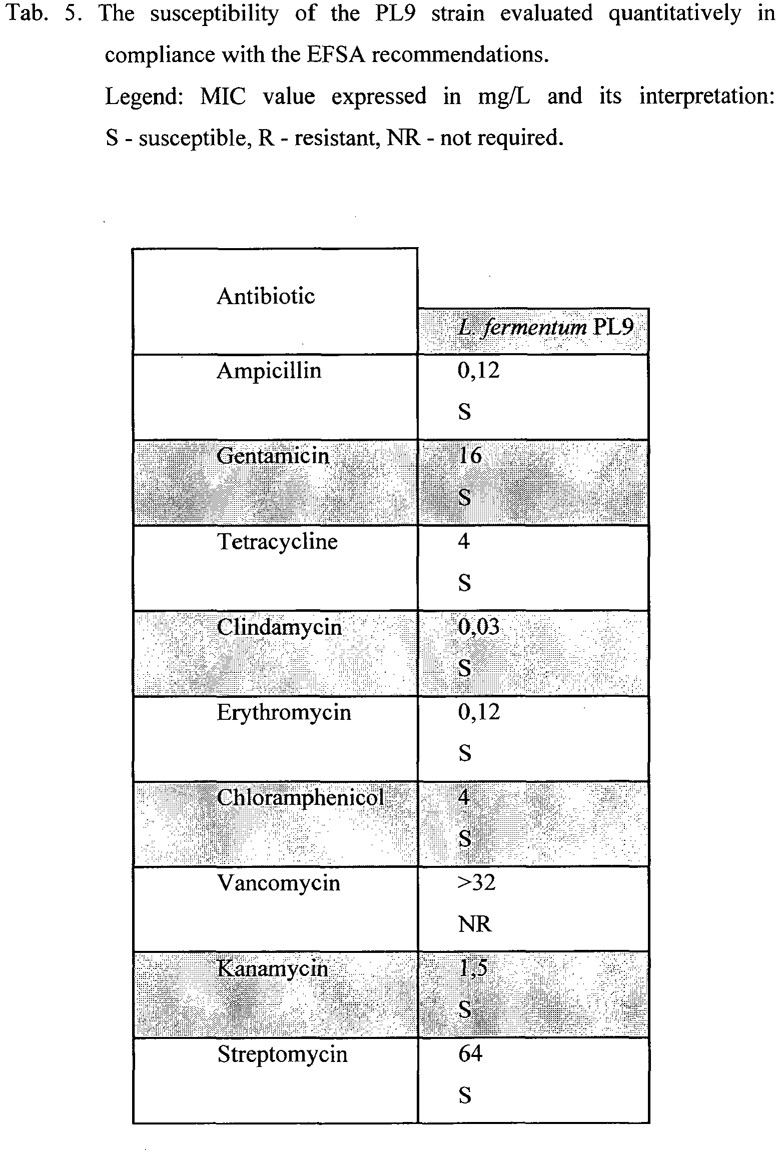

Using the quantitative method it was confirmed that the *Lactobacillus fermentum* PL9 strain shows sensitivity to basic antibiotics consistent with EFSA (European Food Safety Authority) interpretative data.

The resistance of probiotic strains to antibacterial drugs is the subject of detailed studies for the safety of future medicinal preparations. The *Lactobacillus fermentum* PL9 strain shows resistance not different from that typical for this species. This means that it can be safely used in probiotic preparations, because it does not have resistance characteristics acquired through mutation or exchange of genetic material, and thus there is no risk of passing resistance genes to other intestinal bacteria, such as enterococci, which are a natural reservoir of such genes.

### V. Determination of resistance of the Lactobacillus fermentum PL9 strain to artificial gastric juice.

The objective of the study was to determine the survival of the new *Lactobacillus fermentum* PL9 strain in artificial gastric juice. In order to determine the degree of resistance of bacteria of the genus *Lactobacillus* to the pH of gastric juice, the Clark method was used, which was modified in such a way that artificial gastric juice with a pH of 1.2 was applied instead of concentrated hydrochloric acid, according to the following recipe: 2.0 g of NaCl, 3.2 g of pepsin in 7 ml of 36% HCl of pH 1.2 was dissolved in 1000 ml of distilled water. The sterile (after filtration) artificial gastric juice in the amount of 1 ml was poured into sterile tubes, and then 100 µl of fresh (24 hours) culture of the *Lactobacillus fermentum* PL9 strain of known density was added to each of them successively. The mixture was incubated for 30 min. at temperature 37 °C under strictly anaerobic conditions. After the allotted time had elapsed, the mixture was thoroughly mixed and a decimal culture was performed to determine the final density obtained.

**Tab. 5. Survival of the Lactobacillus fermentum PL9 strain in artificial gastric juice.**

| | Density of probiotic bacteria given in CFU/ml | |
|---|---|---|
| Strain name | Output time | After a 30-minute incubation in artificial gastric juice of a pH 1.2 |
| *Lactobacillus fermentum* PL9 | 1,3 x 10⁸ | 3,2 x10⁷ |

The PL9 strain shows high resistance to low pH and the action of artificial gastric juice, which may indicate its perfect adaptation to the unfavorable conditions in the human gastrointestinal tract. This means that this strain will not be inhibited by gastric juice while passing through the digestive system.

### VI. Determination of resistance of the Lactobacillus fermentum PL9 strain to bile salts.

The objective of the study was to determine the resistance of the new *Lactobacillus fermentum* PL9 strain to bile salts. Bile salt resistance was determined based on the method of Dashkevicz and Feighner (Dashkevicz M.P., Feighner S.D. 1989. Development of a differential medium for bile salt hydrolase-active Lactobacillus spp. Applied and Environmental Microbiology. 55(1), 1-16). This method consisted in adding to the MRS or BL agar the dye indicator - bromocresol purple (POCH) in the amount of 0.17 g/l and bile salt (OXGAL, Difco) in five successive concentrations: 1 g/l, 2 g/l, 5 g/l, 10 g/l, 20 g/l. By using the reduction method, 100 µl of the culture of the *Lactobacillus fermentum* PL9 strain was inoculated on this prepared solid substrate, and it was then incubated in standard anaerobic conditions for 48 hours. After the incubation was completed, the colonies of bacteria resistant to a given concentration of bile salt turned light yellow and the growth media changed its color from purple to yellow. The intensity of the growth of the tested strain and the yellow color of the media were assessed on a semi-quantitative scale ranging from - to +++.

**Tab. 6. Resistance of the Lactobacillus fermentum PL9 strain to bile salts in the following concentrations: 1, 2, 5, 10, 20 g/l.**

| Bile salts concentration | *L. fermentum* PL9 |
|---|---|
| 1 g/l | +++ |
| 2 g/l | +++ |
| 5 g/l | +++ |
| 10 g/l | +++ |
| 20 g/l | ++ |

Resistance of the *Lactobacillus fermentum* PL9 strain to bile salts in the concentrations of 1, 2, 5, 10, 20 g/l is shown in Fig. 3.

The PL9 strain shows high resistance to the action of bile salts, which may indicate its perfect adaptation to the unfavorable conditions in the human gastrointestinal tract. This means that this strain will not be inhibited by bile salts during its passage through the digestive system, so it can be used in oral preparations.

### VII. Determination of adherent properties of the Lactobacillus fermentum PL9 strain to the human Caco-2 cell line.

The adherent properties of the *Lactobacillus fermentum* PL9 strain were carried out in relation to the human intestinal epithelial Caco-2 cell line. In order to test the adherence of the tested strain, the intestinal epithelial cells (Caco-2) were cultured in an atmosphere containing 10% CO2 and at a temperature of 37 °C, in DMEM (Gibco) culture medium with the addition of 10% fetal bovine serum (FBS, Sigma-Aldrich). The cultivation time was 15 days. The culture fluids were replaced regularly every 48 hours. After achieving a confluent growth, i.e. "monolayer", cells were passaged with 0.25% Trypsin (Sigma-Aldrich), and then screened on 24-well plates (TPP, Switzerland) bringing them to density of 5 x 10⁵ cells per well. Growing the Caco-2 cell line was continued on the surface of sterile slides placed at the bottom of the 24-well plates for the next 3 days until the confluent growth on the surface of the slides was. Then, the cells were rinsed with PBS without Ca² + and Mg²⁺ ions (IITD PAN; Wroclaw) and 600 µl of appropriately prepared sample were added to each well. The *Lactobacillus fermentum PL9* strain was grown in 10 ml of MRS (Oxoid) liquid medium for 24 hours under anaerobic conditions at temperature 37 °C. After this time, the culture density was evaluated by means of decimal dilutions technique. A total of 100 µl of liquid probiotic bacteria culture was added to each well containing the cultured tissue and 900 µl of fresh DMEM medium was added. The final density of the tested bacteria population was approximately 1x10⁸ CFU in 1 ml. Then the cells of the tissue lines together with the tested strain were incubated at temperature 37° C in an atmosphere with increased humidity and a 10% of CO2 content for 2 hours. After this time, the cells were rinsed with PBS in order to remove non-adherent bacteria, and then the whole was fixed with 3.7% paraformaldehyde at temperature 4 °C for 3 hours. The slides were then washed with PBS and Gram staining was performed. The preparation was assessed under a light microscope at 1000x magnification. The cells of the tested *Lactobacillus fermentum* PL9 strain that adhered to the tissue line were counted in 5 fields of view of the preparation, and then the average result was calculated, which was assigned the appropriate degree of adherence in accordance with the range given in the literature (Tuomola E.M., Salminen S.J. 1998. Adhesion of some probiotic and dairy Lactobacillus strains to Caco-2 cell cultures. Int J Food Microbiol. 41, 45-51):

| | |
|---|---|
| +++ | very high degree of adherence (more than 80 bacterial cells in the field of view) |
| ++ | high degree of adherence (60 to 80 bacterial cells in the field of view) |
| + | moderate degree of adherence (40 to 60 bacterial cells in the field of view) |
| - | poor degree of adherence (less than 40 bacterial cells in the field of view). |

**Tab. 7. Results of the adherence of Lactobacillus fermentum PL9 to the human Caco-2 cell line.**

| Strain name | Degree of adherence | Average number of bacterial cells in the field of view |
|---|---|---|
| *L. fermentum* PL9 | ++ | 60-80 |

| **Repetition number** | ***Lactobacillus fermentum* PL9** | |
|---|---|---|
| | Number of bacterial cells in the field of view | Average of 5 fields of view |
| Repetition I | 60 | 62 |
| | 60 | ++ |
| | 60 | |
| | 60 | |
| | 70 | |
| Repetition II | 60 | 62 |
| | 70 | ++ |
| | 60 | |
| | 60 | |
| | 60 | |
| Repetition III | 50 | 64 |
| | | ++ |

The adherence of the *Lactobacillus fermentum* PL9 strain to the intestinal epithelial Caco-2 cell line is shown in Fig. 4.

The *Lactobacillus fermentum* PL9 strain showed a high degree of adherence to the human intestinal epithelium and thus has the ability to adhere effectively and, consequently, colonize the gastrointestinal tract.

In order for probiotic strains to exert a beneficial effect on the host's health, they must be adhered, which occurs through their binding to appropriate receptors located on the surface of the host's intestinal cells. Tissue adherence enables the development and multiplication of probiotic bacteria, and the colonization of tissues significantly reduces the risk of pathogenic microorganisms invasion/colonization.

### VIII. Determination of the ability of the Lactobacillus fermentum PL9 strain to stimulate the synthesis of tight junction proteins on the example of occludin.

The study was conducted using the Caco-2 cell line. Tissue culture was performed for 20 days in the DMEM medium (IITD, Wroclaw) with the addition of 10% fetal bovine serum (FBS, Sigma-Aldrich) in 24-well culture plates (TPP, Switzerland) in an atmosphere with increased humidity, enriched with 10% CO₂ and at temperature 37 °C. After reaching a confluent monolayer growth (phase), the cells were washed twice with PBS (IITD, Wroclaw) and the evaluation for the synthesis of tight junction proteins between cells of the tested line was performed. A total of 900 µl of fresh DMEM medium without antibiotics and 100 µl of an appropriately prepared culture of the tested probiotic strain (with the addition of 5% MRS agar) were added to each well containing the cultured tissue of an average density of 1x10⁶ cells /ml. Due to the content of 5% MRS agar, the tested probiotic strain adhered firmly to the surface of the Caco-2 cell culture. The co-culture was incubated again for 24 hours in an atmosphere of increased humidity, enriched with 10% CO₂ and at temperature 37 ° C. After this time, the tissue culture was washed 3x with PBS buffer and fixed with methanol at temperature -20 ° C for 2 minutes. Then, 100 µl of FITC labeled monoclonal antibodies detecting tight junction proteins, i.e. occludin (Invitrogen-Corporation), were applied to the surface of each slide (placed in a culture well). Due to the fact that the antibodies binding to the tight junction proteins were stained with the FITC fluorescent dye, it was possible to observe the intensity of the illumination of these junctions in the OLYMPUS BX51 fluorescence microscope at the wavelength of 474 emi. 520 nm. at 400x magnification. The illumination intensity was assessed according to semi-quantitative scale. The assessment was performed from five fields of view and the result was averaged.

The illumination intensity was assessed according to a semi-quantitative scale:
- no illumination (no tight junction proteins formation between cells)
+ low illumination (low level of tight junction protein formation)
++ strong illumination (high level of tight junction protein formation)
+++ very strong illumination (very high level of tight junction protein formation).

**Tab. 8 Stimulation of production of occludin between cells of the Caco-2 cell line after stimulation by the Lactobacillus fermentum PL9 strain.**

| Strain name | Stimulation of occludin production |
|---|---|
| *L. fermentum* PL9 | ++ |

The production of tight junction proteins (occludin) by epithelium after stimulation by the probiotic strain of *Lactobacillus fermentum* PL9 is shown in Fig. 5.

The *Lactobacillus fermentum* PL9 strain has a strong effect on intestinal epithelial cells, stimulating them to produce occludin, which is an example of tight junction proteins. Therefore, the PL9 strain may reduce the permeability of the intestinal epithelium preventing, among others, infections.

### IX. Study of the antagonistic properties of the Lactobacillus fermentum PL9 strain against the etiological factors of gastrointestinal infections.

In order to determine the activity of the tested *L. fermentum* PL9 strain, 900 microliters of 24-hour cultures of the tested strains with an average density of 1x10⁸ CFU/ml was inoculated with 100 microliters of 24-hour cultures of etiological factors of gastrointestinal infections with an average density of 1x10⁶ CFU/ml, prepared by suspending one portion of inoculation loop in: 10 ml of TSB broth (Biocorp) for the following aerobic bacteria: *Staphylococcus aureus* ATCC 25923, *Escherichia coli* ATCC 25922, *Salmonella enteritidis* ATCC 13076; 10 ml Sabouraud broth (Biocorp) for the following yeast-like fungi: *Candida albicans* ATCC 10231, *Candida glabrata* ATCC 15126; 10 ml of Schaedler broth (Biocorp) for the following anaerobic bacteria: *Campylobacter fetus* PCM 2632, *Clostridium difficile* ATCC 17857. The whole co-cultures were incubated in aerobic, microaerophilic and anaerobic conditions, respectively, at temperatures of 37 °C and 41.5 °C. At baseline and after 8 and 24 hours of incubation, decimal dilutions were made from each of the mixtures by inoculating successively 100 microliters of the mixture on Columbia Agar (Biocorp) for the strain of *Staphylococcus aureus* ATCC 25923; MacKonkey Agar (Biocorp) for the strains: *Escherichia coli* ATCC 25922, *Salmonella enteritidis* ATCC 13076; Sabouraud Agar (Biocorp) for the strains: *Candida albicans* ATCC 10231, *Candida glabrata* ATCC 15126; Schaedler Agar (Biocorp): *Campylobacter fetus* PCM 2632, *Clostridium difficile* ATCC 17857. The number of bacterial colonies of the tested pathogenic strains was calculated and the results were given in the colony forming units per 1 ml (CFU/ml).

**Tab. 9. Antagonistic activity of the Lactobacillus fermentum PL9 strain against etiological factors causing gastrointestinal infections based on the quantitative method.**

| Strain | O h CFU/ml | 8 h CFU/ml | 24 h CFU/ml |
|---|---|---|---|
| *Staphylococcus aureus* ATCC 25923 | 1,8x10⁶ | 0 | 0 |
| *Escherichia coli* ATCC 25922 | 1,0x10⁶ | 0 | 0 |
| *Salmonella enteritidis* ATCC 13076 | 1,0x10⁶ | 0 | 0 |
| *Campylobacter fetus* PCM 2632 | 2,0x10⁶ | 0 | 0 |
| *Clostridium difficile* ATCC 17857 | 1,0x10⁶ | 5,6x10⁴ | 0 |
| *Candida albicans* ATCC 10231 | 1,0x10⁶ | 5,0x10⁵ | 0 |
| *Candida glabrata* ATCC 15126 | 2,0x10⁶ | 3,0x10⁵ | 6,0x10³ |
| Control culture *L. fermentum* PL9 | 6,4x10⁸ | 6,0x10⁸ | 5,0x10⁸ |
| Control culture *Staphylococcus aureus* ATCC 25923 | 1,8x10⁶ | 1,0x10⁵ | 2,0x10⁵ |
| Control culture *Escherichia coli* ATCC 25922 | 1,0x10⁶ | 1,2x10⁶ | 1,5x10⁸ |
| Control culture *Salmonella enteritidis* ATCC 13076 | 1,0x10⁶ | 1,1x10⁵ | 1,0x10⁵ |
| Control culture *Campylobacter fetus* PCM 2632 | 2,0x10⁶ | 1,0x10⁶ | 5,0x10⁷ |
| Control culture *Clostridium difficile* ATCC 17857 | 1,0x10⁶ | 1,0x10⁶ | 5,0x10⁷ |
| Control culture *Candida albicans* ATCC 10231 | 1,0x10⁶ | 6,0x10⁷ | 5,0x10⁷ |
| Control culture *Candida glabrata* ATCC 15126 | 2,0x10⁶ | 3,0x10⁶ | 8,0x10⁵ |

The antagonistic activity of the *Lactobacillus fermentum* PL9 strain against selected species of pathogenic bacteria and fungi is shown in Fig. 6.

The PL9 strain shows a remarkably antagonistic effect against all the most common bacterial and fungal pathogenic factors of intestinal infections, except for *C*. *glabrata.* Thus, it can positively affect their elimination from the gut microbiota.

### X. Study of the ability of the Lactobacillus fermentum PL9 strain to form biofilm.

In this study, 96-well polystyrene microplates with an adherence surface (Greiner Bio-One, USA) were used for the biofilm growth of *the Lactobacillus fermentum* PL9 strain. A total of 180 µl of fresh MRS Broth (Oxoid) culture medium and a 20 µl probiotic strain suspension in saline with 0.5 McFarland turbidity were added to each well. The plates were centrifuged (10 minutes, 2000 rpm) in order to get the bacteria settled at the bottom of the culture well, and then placed under anaerobic conditions at temperature 37 °C. The biofilm was cultivated for 48 hours. At appropriate time points, i.e. after 8, 24 and 48 hours of incubation, the number of bacteria was measured using ten-fold dilution and quantitative assessment methods for biofilm growth. Measurements of the biofilm production were carried out using the colorimetric method, after prior staining the contents of the wells with Congo Red in accordance with the quantitative method described by Reuter et al. (Reuter M., Mallett A., Pearson BM, Van Vilet HM 2010. Biofilm formation by Campylobacter jejuni is increased under aerobic conditions Appl. Environ. Microbiol. 76, 2122-2128). The absorbance values in the wells were read at a wavelength of 492 nm using an Awarness Technology INC (USA) spectrophotometer. The numbers of probiotic bacteria at a given time point are presented in Tab. 11. The results of biofilm production by the *L. fermentum* PL9 strain are presented in Fig. 7.

**Tab.10. The number of L. fermentum PL9 bacteria in the biofilm.**

| Strain name | 0 h CFU/ml | 8 h CFU/ml | 24 h CFU/ml | 48 h CFU/ml |
|---|---|---|---|---|
| *L. fermentum* PL9 | 9,5x10⁷ | 3,9x10⁸ | 4,0x10⁸ | 6,0x10⁸ |

### XI. Study of the ability of the Lactobacillus fermentum PL9 strain to adhere to mucin.

The ability of the *Lactobacillus fermentum* PL9 strain to adhere to mucin was tested in accordance with the methodology described by Tallon et al. (Tallon R., Arias S., Bressollier P., Urdaci M.C. 2006. Strain- and matrix-dependent adhesion of Lactobacillus plantarum is mediated by proteinaceous bacterial compounds. Journal of Applied Microbiology. 102, 442-451). For this purpose, mucin (Sigma-Aldrich) was dissolved in buffered saline (PBS, IITD, Wroclaw), so as to obtain a final concentration of 10 mg/ml. A total of 100 µl of the mucin solution prepared in this way was added to microtiter polyester plates (Greiner Bio-One, USA) and immobilized overnight at temperature 4 °C. The wells were then washed twice with 200 µl PBS (Sigma-Aldrich) and a 2% bovine serum albumin solution (BSA, Sigma-Aldrich) was added for 4 hours (temperature of 4 °C). Following incubation, the wells were again rinsed twice with 200 µl PBS (Sigma-Aldrich). The *Lactobacillus fermentum* PL9 strain was grown for 16h in MRS broth (Oxoid) at temperature 37 °C. After the incubation period, 1 ml of the culture was centrifuged (6000 x g for 5 minutes). The supernatant was removed and the cell pellet remaining at the bottom was rinsed twice with 1 ml PBS (Sigma-Aldrich) and adjusted to the optical density (OD) (600 nm) of 0.1-0.01. A total of 100 µl of the bacterial suspension prepared in this way was added to the wells and left for 1h at temperature 37 °C. The wells were then washed 12 times with 200 µl of PBS (Sigma-Aldrich) in order to remove unbound bacteria and treated with 200 µl of 0.5% Triton-X solution (Sigma-Aldrich) to remove the *Lactobacillus fermentum* PL9 bacteria stuck to the bottom of the wells. The plates were further incubated for 2 h at room temperature in an orbital shaker. Nest, a volume of 100 µl of the bacterial suspension was removed from the wells and plated onto MRS Agar (Oxoid). The media was then incubated under anaerobic conditions at temperature 37 °C for 24 hours. Following incubation, the number of bacterial colonies was calculated and the results were given in colony forming units per 1 ml (CFU/ml).

**Tab. 11. Results of mucin adherence ability of the Lactobacillus fermentum PL9 strain.**

| Strain name | Number of bacteria (CFU/ml) | |
|---|---|---|
| | Output time at 0 h | After 1 hour incubation with mucin |
| *L. fermentum* PL9 | 1,0 x 10⁸ | 3,3 x 10⁵ |
| *L. phamnosus GG* | 1,2 x 10⁸ | 5,6 x 10³ |

The new *Lactobacillus fermentum* PL9 strain has been shown to have a high ability to stimulate the production of occludin by epithelial cells - a protein that is an indicator for a whole family of similar tight junction proteins that connect cells and seal the intercellular space of the intestinal epithelium, so that the process of ease movement of substances between the intestinal lumen and epithelial cells and underlying tissues is largely controlled, which thus prevents intestinal permeability and, above all, prevents pathogenic factors from entering the bloodstream.

In addition, the new *Lactobacillus fermentum* PL9 strain has an outstanding ability to form biofilm, so that it strengthens the intestinal barrier and its functions providing defense against pathogenic microorganisms by initiating the formation of a protective layer of bacteria on the surface of the intestinal epithelium. This protective bacteria layer, in turn, prevents the colonization of the intestinal epithelium by pathogenic microorganisms and plays an important role in sealing the intestines. The mechanisms demonstrated by the *Lactobacillus fermentum* PL9 strain mediating biofilm formation are: the formation of an exopolysaccharide layer on the surface of bacterial cells of this strain and the ability to effectively adhere to mucins in the gastrointestinal tract.

The new *Lactobacillus fermentum* PL9 strain shows high resistance to low pH and the action of artificial gastric juice and bile salts, which may indicate its perfect adaptation to the conditions unfavorable for bacteria in the upper parts of the human gastrointestinal tract.

The new *Lactobacillus fermentum* PL9 strain has strong antibacterial and antifungal activities against pathogenic fungi and bacteria (such as *Staphylococcus aureus, Escherichia coli, Salmonella enteritidis, Campylobacter fetus, Clostridium difficile, Candida albicans*), and can therefore be used as an active ingredient of an agent that inhibits and/or limits the growth of the population of pathogenic fungi and bacteria in the human gastrointestinal tract.
## Claims

1. A new *Lactobacillus fermentum* strain designated by the symbol PL9, deposited in accordance with the Budapest Treaty on the International Recognition of the Deposit of Microorganisms for the Purposes of Patent Procedure at the Polish Collection of Microorganisms (PCM), located at the Institute of Immunology and Experimental Therapy in Wroclaw, under the deposit number B/00163.

2. A new *Lactobacillus fermentum* PL9 strain according to claim 1, **characterized in that** it has high ability to stimulate the production of occludin by intestinal cells.

3. A new *Lactobacillus fermentum* PL9 strain according to claim 1, **characterized in that** it has an outstanding ability to form biofilm.

4. A new *Lactobacillus fermentum* PL9 strain according to claim 1, **characterized in that** it has the ability to adhere to mucins of intestinal mucus.

5. Lactobacillus fermentum PL9 deposited at the Polish Microbiological Collection under the deposit number B/00163 for use in the prevention of pathogenic fungi and bacterial infections in the human gastrointestinal tract, by correcting microbiota composition due to pathogens elimination.

6. Lactobacillus fermentum PL9 deposited at the Polish Microbiological Collection under the deposit number B/00163 for use in improving intestinal barrier function.

## Patentansprüche

1. Ein neuer *Lactobacillus Fermentum* stamm mit dem Symbol PL9,
hinterlegt gemäß dem Budapester Vertrag über die internationale Anerkennung der Hinterlegung von Mikroorganismen zum Zwecke des Patentverfahrens bei der Polnischen Sammlung von Mikroorganismen (PCM), die sich im Institut für Immunologie und experimentelle Therapie in Breslau befindet, unter der Hinterlegungsnummer B/00163.

2. Neuer Lactobacillus fermentum PL9-Stamm nach Anspruch 1, **dadurch gekennzeichnet, dass** es eine hohe Fähigkeit besitzt, die Produktion von zu stimulieren Occludin durch Darmzellen.

3. Neuer Lactobacillus fermentum PL9-Stamm nach Anspruch 1, **dadurch gekennzeichnet, dass** es eine hervorragende Fähigkeit zur Biofilmbildung besitzt

4. Neuer Lactobacillus fermentum PL9-Stamm nach Anspruch 1,
**Dadurch gekennzeichnet, dass** es die Fähigkeit besitzt, an Mucinen des Darmschleims zu haften.

5. Lactobacillus fermentum PL9, hinterlegt bei der Polnischen Mikrobiologischen Sammlung unter der Hinterlegungsnummer B/00163 zur Verwendung bei der Vorbeugung von pathogenen Pilzen und bakteriellen Infektionen im menschlichen Magen-Darm-Trakt durch Korrektur der Mikrobiotazusammensetzung aufgrund der Elimination von Krankheitserregern.

6. Lactobacillus fermentum PL9, hinterlegt bei der Polnischen Mikrobiologischen Sammlung unter der Hinterlegungsnummer B/00163 zur Verwendung bei der Verbesserung der Darmbarrierefunktion.

## Revendications

1. Une nouvelle souche de *Lactobacillus fermentum* désignée par le symbole PL9, déposée conformément au Traité de Budapest sur la reconnaissance internationale du dépôt des micro-organismes aux fins de la procédure en matière de brevets auprès de la Collection polonaise de micro-organismes (PCM), située à l'Institut d'immunologie et de thérapie expérimentale de Wroclaw, sous le numéro de dépôt B/00163.

2. Nouvelle souche de *Lactobacillus fermentum* PL9 selon la revendication 1, **caractérisée en ce qu'**elle a une capacité élevée à stimuler la production d'occludine par les cellules intestinales.

3. Nouvelle souche de *Lactobacillus fermentum* PL9 selon la revendication1, **caractérisée en ce qu'**elle a une capacité exceptionnelle à former un biofilm.

4. Nouvelle souche de *Lactobacillus fermentum* PL9 selon la revendication 1, **caractérisée en ce qu'**elle a la capacité d'adhérer aux mucines de la muqueuse intestinale.

5. Lactobacillus fermentum PL9 déposé à la Collection microbiologique polonaise sous le numéro de dépôt B/00163 pour une utilisation dans la prévention des infections fongiques et bactériennes pathogènes dans le tractus gastro-intestinal humain, par la correction de la composition du microbiote due à l'élimination des pathogènes.

6. Lactobacillus fermentum PL9 déposé à la Collection microbiologique polonaise sous le numéro de dépôt B/00163 pour son utilisation dans l'amélioration de la fonction de barrière intestinale.
